Europäisches Patentamt

European Patent Office

Office européen des brevets

(19)

(11) Veröffentlichungsnummer : **0 153 615**
**B1**

(12) **EUROPÄISCHE PATENTSCHRIFT**

(45) Veröffentlichungstag der Patentschrift :
26.04.89

(51) Int. Cl.⁴ : **C 07 D307/60// C07C69/716**

(21) Anmeldenummer : **85101150.2**

(22) Anmeldetag : **04.02.85**

(54) Verfahren zur Herstellung von Tetronsäure.

(30) Priorität : 09.02.84 CH 604/84

(43) Veröffentlichungstag der Anmeldung :
04.09.85 Patentblatt 85/36

(45) Bekanntmachung des Hinweises auf die Patenterteilung : 26.04.89 Patentblatt 89/17

(84) Benannte Vertragsstaaten :
AT BE CH DE FR GB IT LI NL SE

(56) Entgegenhaltungen :
EP—A— 0 067 408
EP—A— 0 067 409
CH—A— 529 128
SYNTHETIC COMMUNICATIONS, Band 11, Heft 5, 1981, New York; D.GROB SCHMIDT et al. "A convenient synthesis of 2,4(3H, 5H)-furandione",Seiten 385-390

(73) Patentinhaber : **LONZA AG**
**Gampel/Wallis (CH)**

(72) Erfinder : **Meul, Thomas, Dr.**
**Balfrinstrasse 21**
**Visp (Kanton Wallis) (CH)**
Erfinder : **Tenud, Leander, Dr.**
**Balfrinstrasse 23**
**Visp (Kanton Wallis) (CH)**
Erfinder : **Huwiler, Alfred, Dr.**
**Sandstrasse 5**
**Visp (Kanton Wallis) (CH)**

(74) Vertreter : **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

**Beschreibung**

Die Erfindung betrifft ein Verfahren zur Herstellung von Tetronsäure aus 2,4-Dichloracetessigestern.

Es ist bekannt, Tetronsäure ausgehend von Acetessigester über den 2,4-Dibromacetessigester und die 3-Bromtetronsäure mit anschliessender Hydrierung mit Natriumamalgan herzustellen [Wolff und Schwabe, Ann. 291 (1896), 231, und W. D. Kumler, J. Am. Chem. Soc. 60 (1938) 863]. Die Ausbeute an Tetronsäure, bezogen auf das Ausgangsmaterial, lag bei 18 %.

Da dieses Verfahren unbefriedigend war, wurden eine ganze Reihe anderer Verfahren entwickelt. Nach CH-PS 503 722 beispielsweise wird 4-Chloracetessigester mit einem aromatischen Amin zum 3-Arylaminocrotolacton umgesetzt und aus diesem durch Behandlung mit Mineralsäuren die Tetronsäure in Freiheit gesetzt. Der Nachteil dieser Methode besteht darin, dass die Isolierung der Tetronsäure nur durch Hochvakuumsublimation zu realisieren ist. Nach CH-PS 529 128 wird von 4-Halogenacetessigsäure ausgegangen, die in wässriger Lösung mit Alkalien umgesetzt wird. Durch Behandeln mit Mineralsäuren wird die Tetronsäure in Freiheit gesetzt. Auch hier muss die Isolierung der Tetronsäure über eine Hochvakuumsublimation erfolgen, ausserdem ist die erzielbare Ausbeute nur 43 bis 44 %.

Nach US-PS 44 21 922 wird zunächst ein 4-Halogenacetessigester in den entsprechenden 4-Benzyloxyacetessigester überführt und aus diesem durch Hydrogenolyse als Zwischenprodukt der entsprechende 4-Hydroxyacetessigester gebildet, der durch Behandeln mit Säure in die Tetronsäure überführt wird. Bei diesem Verfahren kann der 4-Hydroxyacetessigester isoliert werden und nach Isolation die Behandlung mit Säure durchgeführt werden. Es ist aber auch möglich, die Hydrogenolyse in Gegenwart von Säure durchzuführen. Dabei wird der primär in situ gebildete 4-Hydroxyacetessigester unmittelbar in die Tetronsäure überführt. Der Nachteil dieses Verfahrens liegt darin, dass es über aufwendige Zwischenprodukte geführt werden muss. Grob, Schmidt und Zimmer haben dann wieder versucht, die Tetronsäuresynthese ausgehend von Acetessigester über die 3-Bromtetronsäure zu verbessern (Syn. Comm. 11 (5) 1981, 385-390). Dabei wurde die Bromierung des Acetessigesters und die Umsetzung dieses Produktes zu 3-Bromtetronsäure nach W. D. Kumler [J. Am. Chem. Soc. 60 (1938) 859] durchgeführt, die Hydrierung aber mittels $H_2$ und Pd/C vorgenommen. Die Verbesserung liegt in dieser Stufe, wird doch eine Ausbeute von 82 bis 94 % erreicht. Diese Ausbeute ist aber auf die 3-Bromtetronsäure bezogen. Bezieht man sie aber auf die gesamte Reaktion, also einschliesslich der Synthese der 3-Bromtetronsäure aus Acetessigester (nach Kumler etwa 36 %), so liegt sie auch nur bei 29 bis 34 %.

Ein weiterer Nachteil dieses Verfahrens besteht darin, dass infolge anfallender organischer Salze die Tetronsäure nur durch langwierige Extraktion gewonnen werden kann.

Ziel der vorliegenden Erfindung ist es, die Nachteile der bekannten Verfahren zu vermeiden.

Erreicht wird dies erfindungsgemäss durch ein Verfahren gemäss Anspruch 1.

Als 4-Chloracetessigester können prinzipiell alle Ester verwendet werden. Zweckmässig kommen solche von Alkoholen mit 1 bis 6 C-Atomen oder Benzylester in Frage. Vorzugsweise wird der 4-Chloracetessigsäureäthylester verwendet.

Die Chlorierung zum 2,4-Dichlorester kann nach bekannten Methoden durchgeführt werden. Vorzugsweise wird die Chlorierung z. B. mittels Sulfurylchlorid in einem organischen Lösungsmittel oder Lösungsmittelgemisch durchgeführt.

Als Lösungsmittel werden chlorierte aliphatische Kohlenwasserstoffe, wie Methylenchlorid, Chloroform, Tetrachlorkohlenstoff oder aromatische Kohlenwasserstoffe, wie u. a. Toluol, angewendet, vorzugsweise wird aber Methylenchlorid verwendet. Zweckmässig setzt man pro Mol 4-Chloracetessigester 200 bis 1000 g Lösungsmittel ein.

Das Molverhältnis Chlorierungsmittel zu 4-Chloracetessigester beträgt vorzugsweise 1 zu 1.

Die Temperaturen, bei welcher die Chlorierung durchgeführt wird, kann variieren, doch wird zweckmässig bei 0 bis 40 °C gearbeitet.

Der nach der Chlorierung anfallende 2,4-Dichlorester liegt gelöst im Lösungsmittel vor. Vor der Weiterverarbeitung wird dieser 2,4-Dichlorester vorteilhaft vom Lösungsmittel befreit und der thermischen Behandlung unterworfen.

Zweckmässig wird so vorgegangen, dass der 2,4-Dichlorester nach Abzug des Lösungsmittels getrocknet und bei 110 bis 160 °C, vorzugsweise bei 130 bis 150 °C, erhitzt wird. Diese thermische Behandlung wird zweckmässig unter Rückflussbedingungen bei vermindertem Druck oder Normaldruck, vorzugsweise Normaldruck, durchgeführt.

Nach beendeter Reaktion wird die als erstarrtes Produkt vorliegende 3-Chlortetronsäure vorteilhaft mit einem aromatischen Kohlenwasserstoff gereinigt und anschliessend der Hydrierung unterworfen.

Die Hydrierung findet zweckmässig in Wasser statt. Dazu wird die wässrige Lösung der 3-Chlortetronsäure in Gegenwart eines Platinkatalysators, wie Platin, Palladium oder Rhodium, insbesondere in Mengen von 2 bis 10 % auf einem Trägermaterial, wie Bimsstein, Tonerde, Silicagel oder Kohle mit Wasserstoff, unter Druck bei Temperaturen von 0 bis 30 °C hydriert. Vorzugsweise wird mit 5 % Palladium auf Kohle bei Raumtemperatur gearbeitet.

Der Hydrierdruck kann zwischen 3,5 und 20 atm liegen, wobei niedrige Drücke längere Hydrierzeiten bedeuten.

Zweckmässig werden pro Mol 3-Chlortetronsäure 3,0 bis 8,0 g, vorzugsweise 4,0 g, des Palladiumkatalysators verwendet.

Das erfindungsgemässe Verfahren hat gegenüber den bekannten Verfahren des Standes der Technik folgende Vorteile :
· Der 2,4-Dichloracetessigester lässt sich nahezu quantitativ aus dem grosstechnisch verfügbaren 4-Chloracetessigester darstellen und bedarf keiner destillativen Reinigung für die weitere Umsetzung.

Die Ausbeute an 3-Chlortetronsäure (65 bis 75 %) bei der thermischen Cyclisierung des 2,4-Dichloracetessigesters ist doppelt so hoch wie diejenige an 3-Bromtetronsäure aus dem entsprechenden Dibromester.

Bei der Reduktion der 3-Chlortetronsäure zur Tetronsäure kann ohne Zusatz von Basen gearbeitet werden. Somit entfällt eine aufwendige Trennung von Tetronsäure und anorganischen Salzen.

Beispiel 1

335,90 g (2,00 Mol) 4-Chloracetessigsäureäthylester wurden in 1000 ml Methylenchlorid gelöst. Dazu wurden bei Raumtemperatur innerhalb von 2,5 Std 287,12 g (2,04 Mol) Sulfurylchlorid langsam zugetropft. Die Gasentwicklung war nach ca. 10 Std beendet. Anschliessend wurde das Lösungsmittel am Rotationsverdampfer abdestilliert und das gelbe ölige Reaktionsprodukt am Hochvakuum getrocknet.

Man erhielt 405,30 g 2,4-Dichloracetessigsäureäthylester mit einem Gehalt (GC) von 96,1 %.

Der 2,4-Dichloracetessigsäureäthylester wurde 2,5 Std bei 140 °C unter Rückfluss erhitzt. Diese Siedetemperatur erreichte man dadurch, dass die Reaktion unter vermindertem Druck durchgeführt wurde. Da sich der 2,4-Dichloracetessigester während der Reaktion verbrauchte, erhielt man eine konstante Reaktionstemperatur nur durch kontinuierliche Verkleinerung des Druckes (Anfang 90 mbar bis Ende 30 mbar). Die Reaktion war beendet, als kein Rückfluss mehr beobachtet wurde. Man liess das Reaktionsgemisch abkühlen, wobei die Lösung erstarrte. Durch Zugabe von 300 ml Toluol wurde dieses Gemisch wieder rührbar gemacht. Die ausgefallene 3-Chlortetronsäure wurde abgenutscht, mit 500 ml Toluol gewaschen und am Hochvakuum getrocknet. Man erhielt 185,20 g schwach bräunlich gefärbte, mikrokristalline 3-Chlortetronsäure vom Smp. 206 °C, deren Gehalt laut potentiometrischer NaOH-Titration 99,3 % betrug. Dies entsprach einer Ausbeute von 68,4 %, bezogen auf 100 %igen 4-Chloracetessigsäureäthylester. Zur Umkristallisation wurden 170 g 3-Chlortetronsäure in 3400 ml Essigester heiss gelöst und mit 20 g Aktivkohle 2 Std unter Rückfluss gekocht. Die heisse Lösung wurde abfiltriert und die gelblich gefärbte Lösung auf 100 ml eingeengt. Der ausgefallene Niederschlag wurde abgesaugt, mit 100 ml Essigester gewaschen und am Hochvakuum getrocknet. Man isolierte 166 g weisse, mikrokristalline 3-Chlortetronsäure vom Smp. 206 °C mit einem Gehalt von 99,4 %.

Die Mutterlauge der Reaktionslösung wurde eingedampft und am Hochvakuum destilliert.

Aus der ersten Fraktion wurden 12,48 g unumgesetzter 2,4-Dichloracetessigester zurückgewonnen.

50,0 g (0,37 Mol) 3-Chlortetronsäure (Gehalt 99,4 %) wurden in 400 ml Wasser gelöst und mit 1,45 g Palladium auf Kohle 5 % versetzt. Man hydrierte bei einem Anfangsdruck von 5 bar und Raumtemperatur. Nach 7 Std war die Wasserstoffabsorption beendet. Man filtrierte vom Katalysator ab, wusch mit 100 ml Wasser nach und destillierte das Wasser am Rotationsverdampfer bei einer Badtemperatur von maximal 30 °C ab. Die leicht gelbliche Rohsäure wurde 3 Std am Hochvakuum getrocknet.

Ausbeute : 36,5 g weisse kristalline Tetronsäure.

Gehalt (HPLC) 99,2 %.

Die Gesamtausbeute an Tetronsäure, bezogen auf eingesetzten 4-Chloracetessigäthylester betrug 65,6 %.

Beispiel 2

Wie in Beispiel 1 wurde 1 Mol 4-Chloracetessigsäuremethylester mit äquivalenten Mengen Sulfurylchlorid bei Raumtemperatur chloriert. Die Umsetzung zum 2,4-Dichloracetessigsäuremethylester war quantitativ, der Gehalt (GC) betrug 93 % Nachfolgendes 3-stündiges Erhitzen unter Rückfluss bei 140 °C im Vakuum (95 bis 70 mm Hg) lieferte die 3-Chlortetronsäure in 75,5 % Ausbeute, mit einem Gehalt von 100 %.

Die Aufarbeitung erfolgte wie in Beispiel 1. Bei der Destillation der Mutterlauge erhielt man 11,8 % Edukt zurück.

Beispiel 3

Wie in Beispiel 1 wurde 1 Mol 4-Chloracetessigsäurebenzylester mit äquivalenten Mengen Sulfurylchlorid bei Raumtemperatur chloriert. Die Umsetzung zum 2,4-Dichloracetessigsäurebenzylester war quantitativ.

Nachfolgendes 2-stündiges Erhitzen unter Rückfluss bei 130 °C im Vakuum (25 bis 60 mm Hg) lieferte die 3-Chlortetronsäure in 66,3 % Ausbeute, mit einem Gehalt von 99,9 %.

Beispiel 4

Wie in Beispiel 1 wurde 1 Mol 4-Chloracetessigsäureethylester mit äquivalenten Mengen Sulfurylchlorid bei Raumtemperatur chloriert. Die Umsetzung zum 2,4-Dichloracetessigsäureethylester war quantitativ, der Gehalt (GC) betrug 96 %. Nachfolgendes 4-stündiges Erhitzen bei 140 °C bei Normaldruck lieferte die 3-Chlortetronsäure in einer Ausbeute von 84,6 % mit einem Gehalt von 100 %. Nach Weiterumsetzung zur Tetronsäure gemäß Beispiel 1 wurde die Tetronsäure in einer Gesamtausbeute, bezogen auf 4-Chloracetessigsäureethylester, von 74 % erhalten.

**Patentansprüche**

1. Verfahren zur Herstellung von Tetronsäure aus 4-Chloracetessigester, dadurch gekennzeichnet, dass man 4-Chloracetessigester durch Chlorierung in den 2,4-Dichloracetessigester überführt, diesen durch thermische Behandlung bei Temperaturen von 110 bis 160 °C in die 3-Chlortetronsäure überführt und letztere durch Hydrieren mittels Wasserstoff in Gegenwart eines Platinmetallkatalysators in die Tetronsäure überführt.

2. Verfahren nach Patentanspruch 1, dadurch gekennzeichnet, dass man die Chlorierung mittels Sulfurylchlorid bei Temperaturen von 0 bis 40 °C durchführt.

3. Verfahren nach einem oder beiden der Patentansprüche 1-2, dadurch gekennzeichnet, dass man die Hydrierung in Wasser bei Temperaturen von 0 bis 30 °C durchführt.

4. Verfahren nach einem oder mehreren der Patentansprüche 1-3, dadurch gekennzeichnet, dass man die Hydrierung bei einem Hydrierdruck von 3,5 bis 20 atm durchführt.

5. Verfahren nach einem oder mehreren der Patentansprüche 1-4, dadurch gekennzeichnet, dass man als Platinmetallkatalysator Palladium, Platin oder Rhodium in Mengen von 2 bis 10 % auf einem Trägermaterial, wie Bimsstein, Tonerde, Silicagel oder Kohle aufgetragen, verwendet.

**Claims**

1. Process for the preparation of tetronic acid from 4-chloroacetoacetic ester, characterized in that 4-chloroacetoacetic ester is converted into the 2,4-dichloroacetoacetic ester by chlorination, the latter is converted into 3-chlorotetronic acid by thermal treatment at temperatures of 110 to 160 °C, and the latter acid is converted into tetronic acid by hydrogenation with hydrogen in the presence of a platinum metal catalyst.

2. Process according to patent claim 1, characterized in that the chlorination is effected with sulfuryl chloride at temperatures of 0 to 40 °C.

3. Process according to one or both of patent claims 1 to 2, characterized in that the hydrogenation is effected in water at temperatures of 0 to 30 °C.

4. Process according to one or more of patent claims 1 to 3, characterized in that the hydrogenation is effected at a hydrogenation pressure of 3.5 to 20 at.

5. Process according to one or more of patent claims 1 to 4, characterized in that as platinum metal catalyst palladium, platinum or rhodium is used in amounts of 2 to 100 %, deposited on a support material, such as pumice, alumina, silica gel or carbon.

**Revendications**

1. Procédé pour la préparation de l'acide tétronique à partir d'un ester de l'acide 4-chloroacétylacétique, caractérisé en ce qu'on transforme un ester de l'acide 4-chloroacétylacétique par chloration, en l'ester de l'acide 2,4-dichloroacétylacétique, on transforme celui-ci par traitement thermique à des températures de 110 à 160°c en acide 3-chlorotétronique et on transforme ce dernier par hydrogénation au moyen d'hydrogène en présence d'un catalyseur métallique de type platine en acide tétronique.

2. Procédé selon la revendication 1, caractérisé en ce que la chloration est effectuée au moyen de chlorure de sulfuryle à des températures de 0 à 40 °C.

3. Procédé selon l'une ou les deux des revendications 1-2, caractérisé en ce qu'on effectue l'hydrogénation dans l'eau à des températures de 0 à 30 °C.

4. Procédé selon l'une ou plusieurs des revendications 1-3, caractérisé en ce qu'on effectue l'hydrogénation sous une pression d'hydrogénation de 3,5 à 20 atm.

5. Procédé selon l'une ou plusieurs des revendications 1-4, caractérisé en ce qu'on utilise, en tant que catalyseur métallique de type platine, le palladium, le platine ou le rhodium déposé en des quantités de 2 à 10 % sur un matériau de support tel que la pierre ponce, l'alumine, un gel de silice ou du charbon.